# EUROPEAN PATENT SPECIFICATION

(11) **EP 2 575 798 B1**
(45) Date of publication and mention of the grant of the patent: **09.08.2017**
(21) Application number: 11731070.6
(22) Date of filing: 01.06.2011
(51) Int. Cl.: A61K 31/197, A61K 47/32, A61K 47/38, A61K 47/44, A61K 9/00, A61K 9/20, A61K 31/195

(54) **GASTRORETENTIVE DOSAGE FORMS OF GABA ANALOGS**
GASTRORETENTIVE DOSIERUNGSZUBEREITUNG VON GABA ANALOGA
FORMES GALÉNIQUES GASTRORÉTENTIVES D'ANALOGUES DE GABA

(30) Priority: 01.06.2010 IN 1676MU2010
(43) Date of publication of application: 10.04.2013
(73) Proprietor: Rubicon Research Private Limited, Mumbai 400 078 Maharashtra (IN)
(72) Inventor: PILGAONKAR, Pratibha, Sudhir, Bhandup (West) Mumbai 400 078 Maharashtra (IN); RUSTOMJEE, Maharukh, Tehmasp, Bhandup (West) Mumbai 400 078 Maharashtra (IN); GANDHI, Anilkumar, Surendrakumar, Bhandup (West) Mumbai 400 078 Maharashtra (IN)
(74) Representative: HGF Limited
(86) International application number: PCT/IB2011/001200
(87) International publication number: WO 2011/151708

(56) References cited:
- WO-A1-03/103634
- WO-A1-2009/084040
- WO-A1-2010/064100
- WO-A1-2010/143052
- WO-A2-2007/079195
- WO-A2-2011/053003
- US-A- 6 120 803
- US-B2- 7 438 927
- JIVANI R R ET AL: "Design and development of a self correcting monolithic gastroretentive tablet of baclofen", SCIENTIA PHARMACEUTICA 2009 OSTERREICHISCHE APOTHEKER-VERLAGSGESELLSCHAFT M.B.H. AUT LNKD- DOI:10.3797/SCIPHARM.0905-11, vol. 77, no. 3, 2009, pages 651-667, XP002655157, ISSN: 0036-8709
- ANONYMOUS: "EUDRAGIT RL PO", , 3 May 2010 (2010-05-03), XP055293850, Retrieved from the Internet: URL:https://web.archive.org/web/2010050301 4735/http://eudragit.evonik.com/product/eu dragit/en/products-services/eudragit-produ cts/sustained-release-formulations/rl-po/p ages/default.aspx [retrieved on 2016-08-05]
- ANONYMOUS: "EUDRAGIT RS PO", , 30 April 2010 (2010-04-30), XP055293851, Retrieved from the Internet: URL:https://web.archive.org/web/2010043005 5358/http://eudragit.evonik.com/product/eu dragit/en/products-services/eudragit-produ cts/sustained-release-formulations/rs-po/p ages/default.aspx [retrieved on 2016-08-05]
- ANONYMOUS: "EUDRAGIT NE 30 D", , 30 April 2010 (2010-04-30), XP055293854, Retrieved from the Internet: URL:https://web.archive.org/web/2010043005 5818/http://eudragit.evonik.com/product/eu dragit/en/products-services/eudragit-produ cts/sustained-release-formulations/ne-30-d /pages/default.aspx [retrieved on 2016-08-05]

## Description

### FIELD OF THE INVENTION

The present invention relates to gastroretentive dosage forms of gamma aminobutyric acid ("GABA") analogs, and to processes for preparation of the same. Particularly, the invention relates to gastroretentive dosage forms comprising GABA analog, at least one swelling agent and at least one non-swelling release retardant.

### BACKGROUND OF THE INVENTION

Gamma aminobutyric acid ("GABA") is a principal inhibitory neurotransmitter in the central nervous system of mammals. GABA regulates neuronal excitability by binding to specific transmembrane receptors (Cl⁻-channel-coupled GABA_{A} receptors and G-protein-coupled GABA_{B} receptors) resulting in stabilization or hyperpolarization of the resting membrane potential. Attenuation of GABAergic neurotransmission is involved in the pathophysiology of several central nervous system disorders in humans, namely anxiety, epileptic seizures, movement disorders, panic, depression, alcoholism, pain and manic behavior. Numerous GABA analogs have therefore been synthesized and described in the art. Amongst the synthesized GABA analogs, gabapentin, pregabalin, vigabatrin and baclofen have been marketed and used for the treatment of different disorders.

1-(Aminomethyl)cyclohexaneacetic acid, called gabapentin, is a pharmacological agent that mimics the effects of GABA (y-aminobutyric acid), but does not appear to bind a GABA receptor (e.g., GABA_{A} and GABA_{B} receptors) or have an effect on GABA uptake. Gabapentin has been found to interact with the alpha-2-delta subunit of voltage-gated calcium channels on neurons in the central nervous system, decreasing calcium ion flow into a neuron and rendering the neuron less excitable. Thus, like GABA, gabapentin can dampen overactive neural circuitry. Gabapentin disclosed in U.S. Patents 4,024,175 and 4,087,544, marketed under the name NEURONTIN® in the U.S is used for the treatment of diseases such as epilepsy, faintness, hypokinesia as well as cranial traumas. U.S. Patent 5,084,479 discloses that gabapentin is used for the treatment of neurodegenerative disorders such as Alzheimer's disease, Huntington's chorea or Parkinson's disease and amyotrophic lateral sclerosis. U.S. Patent 5,025,035 discloses that gabapentin is used for the treatment of depression. U.S. Patent 5,510,381 discloses that this compound is used for the treatment of mania and bipolar disorder. Gabapentin is freely soluble in water and in acidic and basic aqueous solutions. Elimination half-life of gabapentin is 5 to 7 hours. It is currently available in 100mg, 300mg and 400mg hard shell capsule as well as 600mg and 800mg tablet dosage forms, with recommended dosing of 900mg to 1800mg total daily dose in three divided dosages.

5-methyl-3-aminomethyl-hexanoic acid, called pregabalin is an analog of GABA that decreases central neuronal excitability by binding to an auxiliary alpha-2-delta subunit of a voltage-gated calcium channel on neurons in the central nervous system. Pregabalin, disclosed in U.S. Patents 5,563,175 and 6,197,819, marketed under the name LYRICA® in the U.S is used in the treatment of peripheral neuropathic pain, epilepsy and generalized anxiety disorder. Pregabalin is also effective at treating chronic pain in disorders such as fibromyalgia and spinal cord injury. U.S. Patent 6,117,906 discloses the use of pregabalin in treating anxiety; U.S. Patent 6,001,876 discloses the use of pregabalin in treating pain; U.S. Patent 6,127,418 discloses the use of pregabalin in treating gastrointestinal damage. PCT Publication WO98/58641 discloses use of pregabalin as an anti-inflammatory agent. Pregabalin is freely soluble in water and in basic and acidic aqueous solutions. Elimination half-life of pregabalin is about 6.3 hours. It is available as an immediate release formulation in capsules and is administered two- or three-times daily.

Frequent dosing using immediate release formulations is generally associated with poor patient compliance. A once or twice daily sustained release dosage form of GABA analog would enable better compliance and offer advantages over conventional immediate release formulations. Once or twice per day dosing would lessen or prevent potentially undesirable dose-related effects by reducing peak blood levels (Cmax) and may also increase drug efficacy by increasing minimum plasma concentrations (Cmin).

Conventional extended or sustained release once or twice daily dosing compositions however present numerous challenges in case of GABA analogs. The oral bioavailability of gabapentin is dose-dependent, with approximately 60% bioavailability for a dose in range of 300-400mg, but with only 35% bioavailability for a dose of 1600mg. The decrease in bioavailability with dose has been attributed to carrier-mediated absorption. Following oral administration, gabapentin is absorbed from the small intestine which occurs via a specific, amino acid transporter mechanism that becomes saturated at higher doses. Saturation of this transporter at doses used clinically leads to dose-dependent pharmacokinetics and high interpatient variability. A conventional sustained or extended release dosage form may not be useful in this case. Since gabapentin is absorbed high in the gastrointestinal tract, by means of a saturable transport mechanism, a gastric retained dosage form is particularly beneficial for delivery of gabapentin because such a dosage form can release the drug in the region of absorption, show improved bioavailability and avoid saturation of the carrier mediated transport. Further pregabalin too is not absorbed uniformly in the gastrointestinal tract. Pregabalin is absorbed in the small intestine and the ascending colon in humans via amino acid transporters, but is poorly absorbed beyond the hepatic flexure. In such instances therefore again a convnetional extended release or sustained release dosage form may not be useful as any drug release occurring after the dosage form has traveled beyond the hepatic flexure may be of no use. A sustained release gastroretentive dosage form may therefore be an ideal dosage form in such instances.

Attempts have been made by researchers to provide gastroretentive systems of GABA analogs. U.S. Patent Application 2007/0269511 relates to a pharmaceutical composition comprising pregabalin, and matrix forming agent and a swelling agent, the matrix-forming agent comprising polyvinyl acetate and polyvinylpyrrolidone, and the swelling agent comprising cross-linked polyvinylpyrrolidone, wherein the pharmaceutical composition is adapted for once-daily oral dosing. U.S. Patent 6,723,340 discloses a controlled release tablet comprising a solid monolithic matrix with gabapentin dispersed therein, with said matrix comprising a combination of poly (ethylene oxide) and hydroxypropyl methylcellulose at weight ratio that causes said matrix to swell upon contact with gastric fluid to a size large enough to provide gastric retention. U.S. Patent 7,438,927 discloses a method of treating epilepsy comprising administering gabapentin dispersed in a gastric retained dosage form, wherein the dosage form comprises a single polymer matrix comprising at least one swellable hydrophilic polymer that swells in a dimensionally unrestrained manner by imbibing water to increase its size to promote gastric retention of the dosage form in the stomach of the mammal. U.S. Patent Application 2004/0180088 discloses a gastric retentive controlled drug delivery system comprising baclofen or its pharmaceutically acceptable salt, a highly swellable polymer and a gas generating agent, and said system capable of swelling and achieving floatation rapidly. PCT Publication WO2010/143052A1 discloses a gastroretentive floating tablet of pregabalin comprising one or more water insoluble components preferably a combination of ethyl cellulose and hydrogenated castor oil. PCT Publication WO2011/053003A2 discloses a gastric-retentive sustained release formulation containing pregabalin or pharmaceutically acceptable salts thereof, polyethylene oxide, and polyvinyl alcohol-polyethylene glycol graft copolymer.

Most of the above patents/patent applications disclose gastroretentive systems, which utilize mainly hydrophilic swelling agents. However for highly soluble drugs such as GABA analogs, use mainly of matrices based on such swelling agents does not provide adequate control over the drug release rate, instead resulting in a release that approximates first order kinetics. A need thus exists for the development of gastroretentive systems for delivery of GABA analogs that are not based just on the use of hydrophilic swelling agents but incorporate excipients that help offer the desired control over the drug release rate and also provide adequate gastroretention.

Further a significant problem of GABA analogs is the formation of toxic impurities such as the corresponding gamma-lactams during synthesis and/or formulation and/or storage. The amino group of GABA analogs reacts with its carboxyl functional group to form lactams and it needs to be minimized for safety reasons. GABA analogs under usual storage conditions and also in the presence of water tend to form the undesirable lactam side product. Many of the excipients that may be used for formulating preparations of GABA analogs tend to react with them with lapse of time to form the corresponding lactams by accelerating the dehydration reaction between the amino group and the carboxyl group within the GABA analog molecule. In the case of gabapentin, the intramolecular lactam 4-cyclohexylpyrrolidone is considered to be more toxic than gabapentin. The cyclic lactam of pregabalin (4-isobutyl-pyrrolidin-2-one) is also an undesired side product. Hence controlling lactam impurity during development and shelf life of pharmaceutical compositions of GABA analog is an important parameter. Further, the primary amino-group present in the GABA molecule not only is able to form a lactam-ring but also to react with other reducing carbonyl functions. With excipients such as lactose, pregabalin is also known to form conjugates by undergoing a Maillard reaction. It is therefore desirable to provide pharmaceutical compositions that do not comprise conjugate forming excipients, thereby being essentially free of such conjugates and ensuring stability under storage conditions.

Thus, there exists a need to develop stable gastroretentive dosage forms of GABA analogs that provide adequate control over drug release and gastric residence time, and provide desired bioavailability of the active.

The present inventors after thorough research provide gastroretentive dosage forms of GABA analogs comprising GABA analog, at least one swelling agent and at least one non-swelling release retardant.

### SUMMARY OF THE INVENTION

In a first aspect of the present invention is provided a gastroretentive dosage form comprising a GABA analog, at least one swelling agent, at least one non-swelling pH dependent polymeric release retardant and at least one pharmaceutically acceptable excipient;
wherein the GABA analog is selected from pregabalin, gabapentin, baclofen, vigabatrin, ((1R,5S)-3-Aminomethyl-1,5-dimethyl-bicyclo[3.2.0]hept-3-yl)-acetic acid; ((1S,5R)-3 -Aminomethyl-1,5-dimethyl-bicyclo [3.2.0]hept-3 -yl)-acetic acid; ((1R,5S)-3-Aminomethyl-6,6-dimethyl-bicyclo[3.1.0]hex-3-yl)-acetic acid; ((1S,5R)-3-Aminomethyl-6,6-dimethyl-bicyclo[3.1.0]hex-3-yl)-acetic acid; ((1S,2S,5R)-2-Aminomethyl-6,6-dimethyl-bicyclo[3.1.0]hex-2-yl)-acetic acid; ((1R,2S,5S)-2-Aminomethyl-6,6-dimethyl-bicyclo[3.1.0]hex-2-yl)-acetic acid; ((1S,2R,5R)-2-Aminomethyl-6,6-dimethyl-bicyclo[3.1.0]hex-2-yl)-acetic acid; ((1R,2R,5S)-2-Aminomethyl-6,6-dimethyl-bicycIo[3.1.0]hex-2-yl)-acetic acid; ((1R,5R,6S)-6-Aminomethyl-bicyclo[3.2.0]hept-6-yl)-acetic acid; ((1S,5S,6S)-6-Aminomethyl-bicyclo[3.2.0]hept-6-yl)-acetic acid; ((1R,5R,6R)-6-Aminomethyl-bicyclo[3.2.0]hept- 6-yl)-acetic acid; ((1S,5S,6R)-6-Aminomethyl-bicyclo[3.2.0]hept-6-yl)-acetic acid; cis- ((1S,2R,4S,5R)-3-Aminomethyl-2,4-dimethyl-bicyclo[3.2.0]hept-3-yl)-acetic acid; trans-((1S,2R,4S,5R)-3-Aminomethyl-2,4-dimethyl-bicyclo[3.2.0]hept-3-yl)-acetic acid; ((1S,5R,6S,7R)-3-Aminomethyl-6,7-dimethyl-bicyclo[3.2.0]hept-3-yl)-acetic acid; ((1S,5R,6R,7S)-3-Aminomethyl-6,7-dimethyl-bicyclo[3.2.0]hept-3-yl)-acetic acid; ((1R,2S,5S)-7-Aminomethyl-3,3-dimethyl-tricyclo[3.3.0.0]oct-7-yl)-acetic acid; ((1R,6R,7S)-7-Aminomethyl-bicyclo[4.2.0]oct-7-yl)-acetic acid; ((1S,6S,7S)-7-Aminomethyl-bicyclo[4.2.0]oct-7-yl)-acetic acid; ((1R,6R,7R)-7-Aminomethyl- bicyclo[4.2.0]oct-7-yl)-acetic acid; ((1S,6S,7R)-7-Aminomethyl-bicyclo[4.2.0]oct-7-yl)-acetic acid; ((1R,7R,8S)-8-Aminomethyl-bicyclo[5.2.0]non-8-yl)-acetic acid; ((1S,7S,8S)-8-Aminomethyl-bicyclo[5.2.0]non-8-yl)-acetic acid; ((1R,7R,8R)-8-Aminomethyl-bicyclo[5.2.0]non-8-yl)-acetic acid; ((1S,7S,8R)-8-Aminomethyl-bicyclo[5.2.0]non-8-yl)-acetic acid. [(1R,5R,6S)-6-(Aminomethyl)bicyclo[3.2.0]hept-6-yl]acetic acid; [(1S,5S,6R)-6-(Aminomethyl)bicyclo[3.2.0]hept-6-yl]acetic acid; (1RS,5RS,6RS)-6-(Aminomethyl)bicyclo[3.2.0]hept-6-yl]acetic acid; and [(1RS,6RS,7SR)-7-(Aminomethyl)bicyclo[4.2.0]oct-7-yl]acetic acid or a combination thereof;
wherein the amount of the swelling agent in the dosage form is from 5% to 70% by weight of the dosage form, and wherein the swelling agent(s) swell voluminously in the presence of gastric contents to increase the dosage form size such that its passage through the pyrolus is precluded; and
wherein the non-swelling pH dependent polymeric release retardant is present in the formulation in an amount from 2% to 85% by weight of the dosage form and wherein the non-swelling pH dependent polymeric release retardant is polymethacrylic acid polymer, or a derivative thereof selected from methyl acrylate' acrylic acid copolymer, methyl acrylate'methacrylic acid copolymer, methacrylic acid'ethyl methacrylate copolymer, methacrylic acid'ethyl acrylate copolymer, methyl acrylate methacrylic acid octyl acrylate copolymer or a combination thereof.

### DETAILED DESCRIPTION OF THE INVENTION

Pharmaceutical dosage forms that are retained in the stomach for a prolonged period of time after oral administration and release the active ingredient in a sustained manner are ideal for delivering GABA analogs. Design of such specialized dosage forms is a challenge for a formulator because of the complexities of physiological effects that have implications on drug release and absorption in vivo.

The present inventors after rigorous experimentation for the selection of optimum swelling agents to achieve a size of the dosage form that precludes passage through pyloric sphincter to permit gastroretention and non-swelling release retardants to provide desired in-vitro dissolution profiles have designed controlled release or sustained release gastroretentive dosage form of GABA analog for once or twice a day administration. The gastroretentive sustained release dosage forms of GABA analogs of the present invention comprise GABA analog, at least one swelling agent and at least one non-swelling release retardant.

The term "composition" or "formulation" or "dosage form" has been employed interchangeably for the purpose of the present invention and mean that it is a pharmaceutical composition which is suitable for administration to a subject. For the purpose of the present invention the terms "controlled release" or "sustained release" or "extended release" have been used interchangeably and mean that the dosage form is designed to release GABA analog over an extended period of time e.g. from about 1 to about 24 hours; from about 2 to about 24 hours; from about 2 hours to about 20 hours; from about 4 to about 16 hours; from about 4 to about 12 hours; so that therapeutically beneficial levels of GABA analog are maintained over an extended period of time, e.g. from about 2 to about 24 hours; from about 4 to about 24 hours; from about 4 to about 20 hours; from about 4 to about 16 hours; from about 4 to about 12 hours. The subject can be an animal, preferably a mammal, more preferably a human. For the purpose of the present invention the term "gastroretentive" or "gastric retention" or "gastroretention" or "retained in upper gastrointestinal tract" when used in connection with the dosage form of the present invention, means that at least a portion of the dosage form remains in the upper gastrointestinal tract including stomach, for about more than 30 minutes. The sustained release gastroretentive dosage forms of the present invention can be administered once or twice daily.

A GABA analog, in the form of, but not limited to, an acid, a pharmaceutically acceptable salt, a solvate, a polymorph, a prodrug, a hydrate, an enantiomer, an optical isomer, a tautomer, a racemic mixture or a derivative thereof, is employed in the present invention. In one embodiment the GABA analog is pregabalin or a pharmaceutically acceptable salt, a solvate, a polymorph, a prodrug, a hydrate, an enantiomer, an optical isomer, a tautomer, a racemic mixture or a derivative thereof. In another embodiment the GABA analog is gabapentin or a pharmaceutically acceptable salt, a solvate, a polymorph, a prodrug, a hydrate, an enantiomer, an optical isomer, a tautomer, a racemic mixture or a derivative thereof.

A pharmaceutically effective amount of GABA analog in the form of, but not limited to, an acid, a pharmaceutically acceptable salt, a solvate, a polymorph, a prodrug, a hydrate, an enantiomer, an optical isomer, a tautomer, a racemic mixture or a derivative thereof is employed in the formulations of the present invention. The term "effective amount" refers to an amount effective to achieve desired therapeutic and/or beneficial effect. Therapeutic or beneficial effect may be desired in various conditions such as epilepsy, faintness attacks, hypokinesia, cranial traumas, neurodegenerative disorders such as Alzheimer's disease, Huntington's chorea or Parkinson's disease and amyotrophic lateral sclerosis, depression, mania and bipolar disorders, anxiety, panic inflammation, renal colic, insomnia, gastrointestinal damage, incontinence, pain including neuropathic pain, muscular pain, skeletal pain and migraine.

The GABA analog is present in the compositions of the present invention in a pharmaceutically effective amount of about 1% to about 95% by weight of the composition. In a further embodiment, GABA analog is present in the compositions of the present invention in a pharmaceutically effective amount of about 2% to about 90% by weight of the composition In another embodiment, GABA analog is present in the compositions of the present invention in a pharmaceutically effective amount of about 5% to about 85% by weight of the composition According to the present invention, pregabalin is present in the pharmaceutical compositions of the present invention in an amount of about 1% to about 95% by weight of the composition. In one embodiment, pregabalin is present in the compositions of the present invention in an amount of about 2% to about 90% by weight of the composition. In a further embodiment, pregabalin is present in the compositions of the present invention in an amount of about 5% to about 85% by weight of the composition. According to the present invention, gabapentin is present in the pharmaceutical compositions of the present invention in an amount of about 1% to about 95% by weight of the composition. In one embodiment, gabapentin is present in the compositions of the present invention in an amount of about 2% to about 90% by weight of the composition. In a further embodiment, gabapentin is present in the compositions of the present invention in an amount of about 5% to about 85% by weight of the composition. GABA analog in the form of, but not limited to, powder, granules, pellets, beads, minitablets or the like can be employed in the compositions of the present invention. In one embodiment, pregabalin in the form of, but not limited to, powder, granules, pellets, beads, minitablets or the like can be employed in the compositions of the present invention. In another embodiment, gabapentin in the form of, but not limited to, powder, granules, pellets, beads, minitablets or the like can be employed in the compositions of the present invention.

The gastroretentive dosage forms of the present invention comprising GABA analog further comprise at least one swelling agent. The swelling agents employed swell voluminously in the presence of gastric contents to increase the dosage form size such that its passage through the pyrolus is precluded and the dosage form is retained in the upper gastrointestinal tract.

The swelling agent employed in the gastroretentive dosage forms of the present invention includes, but is not limited to, one or more swellable biocompatible hydrophilic polymers. In one embodiment, the swelling agents are employed in the dry state or in a form that has substantial capacity for water uptake. Hydrophilic polymers used as swelling agents in the compositions of the present invention are polymers that are nontoxic and swell in a dimensionally unrestricted manner upon imbibing gastric fluid. Suitable swelling agents employed in the dosage forms of the present invention include, but are not limited to, polyalkylene oxides, cellulosic polymers, acrylic acid polymers, maleic anhydride polymers; polymaleic acid, poly(acrylamides), poly(olefinic alcohol)s, poly(N-vinyl lactams), polyols, polyoxyethylated saccharides, polyoxazolines, polyvinylamines, polyvinyl alcohol, polyimines, polysaccharides, polyurethane hydrogels, zein, shellac-based polymers or derivatives or mixtures thereof.

The cellulosic polymers and derivatives thereof employed in the composition of the present invention include, but are not limited to, methylcellulose, hydroxymethyl cellulose, hydroxypropylcellulose, hydroxyethylcellulose, hydroxypropylmethylcellulose, calcium carboxymethyl cellulose and sodium carboxymethylcellulose. In terms of their viscosities, in one embodiment cellulosic polymers and derivatives thereof employed as swelling agent in the dosage forms of the present invention include those with viscosity within the range of about 50 centipoise to about 200000 centipoise as a 2% aqueous solution at 20° C. In another embodiment, cellulosic polymers and derivatives thereof employed as swelling agent in the dosage forms of the present invention include those with low viscosity of within the range of about 50 centipoise to about 15,000 centipoise as a 2% aqueous solution at 20°C. In a further embodiment, the cellulosic polymers and derivatives thereof employed as swelling agent in the dosage forms of the present invention include those with high viscosity of within the range of about 20,000 centipoise to about 2,00,000 centipoise as a 2% aqueous solution at 20° C.

The polyalkylene oxides and derivatives thereof employed in the compositions of the present invention include, but are not limited to, polyethylene oxide. In one embodiment, polyethylene oxide polymers employed have molecular weights of about 4,000,000 and higher. In one embodiment, polyethylene oxide polymers with molecular weights within the range of about 4,000,000 to about 10,000,000 are employed. In another embodiment, polyethylene oxide polymers with molecular weights within the range of about 4,500,000 to about 9,000,000 are employed. In a further embodiment, polyethylene oxide polymers employed have viscosity in range of about 50 to about 2,000,000 centipoise for a 2% aqueous solution at 20° C.

The polysaccharides and derivatives thereof employed in the compositions of the present invention include, but are not limited to, starch and starch-based polymers e.g. pre-gelatinized starch; chitosan; alginates; maltodextrin; polysaccharide gums such as, but not limited to, xanthan gum, guar gum, locust bean gum, fenugreek gum, galactomannans, gellan, konjac, guar gum, inulin, karaya gum; and the like or combinations thereof.

In one embodiment the swelling agents employed in the compositions of the present invention include, but are not limited to, polyethylene oxide, hydroxypropyl cellulose, hydroxypropyl methyl cellulose, hydroxyethyl cellulose, sodium carboxymethyl cellulose, calcium carboxymethyl cellulose, methyl cellulose, polyacrylic acid, xanthan gum, polyvinyl alcohol or mixtures thereof.

The amount of the swelling agent in the dosage form of the present invention is about 5% to about 70% by weight of the dosage form. In an embodiment, the amount of the swelling agent in the dosage form of the present invention is about 15% to about 50% by weight of the dosage form. In a further embodiment, the amount of the swelling agent in the dosage form of the present invention is about 10% to about 95% by weight of the dosage form. In another embodiment, the amount of the swelling agent in the dosage form of the present invention is about 20% to about 95% by weight of the dosage form. In one embodiment, the amount of the swelling agent in the dosage form of the present invention is about 30% to about 95% by weight of the dosage form.

The amount and type of swelling agents employed in the dosage forms of the present invention ensures that there is sufficient swelling for retention of the dosage form. These swelling agents ensure that within 2 hours at least two dimensions of the dosage form namely length and width is more than 10 mm and cause gastroretention of the dosage form.

The gastroretentive dosage forms of the present invention in addition to GABA analog and at least one swelling agent further comprise at least one non-swelling release retardant. The term "non-swelling release retardant" as used herein refers to any excipient that retards the release of an active pharmaceutical ingredient and that does not swell in water or swells only moderately. This definition does not include excipients such as super disintegrants and polymers such as polyethylene oxide that swell voluminously in contact with water or aqueous media.

According to the present invention, the non-swelling polymeric release retardant is a non-swelling pH dependent polymeric release retardant. Non-swelling polymeric release retardants that are pH dependent exhibit pH dependent solubility, and hence their performance depends on the pH of the environment they encounter. Non-swelling polymeric release retardants that are pH independent exhibit solubility that is independent of pH and hence its performance does not depend on the pH of the environment they encounter. The non-swelling polymeric release retardants employed in the compositions of the present invention along with swelling agents tend to provide lower variability and better control over the release of water-soluble active agents as compared to polymeric systems comprising only swelling polymers.

Non-swelling pH dependent polymeric release retardant is employed in the composition of the present invention. Non-swelling pH dependent polymeric release retardants employed includemethyl acrylate' acrylic acid copolymer, methyl acrylate' methacrylic acid copolymer, methacrylic acid' methyl methacrylate copolymer {e.g., Eudragit® L 100 and Eudragit® S, available from Rohm Pharma), methacrylic acid'ethyl acrylate copolymer (e.g., Eudragit® L 100-55, available from Rohm Pharma), methyl acrylate'methacrylic acid'octyl acrylate copolymer and combinations thereof.

The gastroretentive dosage forms of the present invention comprise GABA analog, at least one swelling agent and at least one non-swelling pH-dependent polymeric release retardant. Without being bound to any theory, the use of at least one non-swelling pH-dependent polymeric release retardant with at least one swelling agent in the gastroretentive dosage form of the present invention helps reduce the initial burst release of highly soluble GABA analogs in the upper gastrointestinal tract than that observed from conventional gastroretentive dosage forms comprising only swelling agents and also helps reduce excessive drug release retardation that may be observed with conventional gastroretentive dosage forms comprising only swelling agents after the gastroretentive dosage form passes further down from the upper gastrointestinal region

The amount of non-swelling release retardant employed in the formulation of the present invention may vary depending upon the degree of controlled release desired. The non-swelling release retardant is present in the formulation in an amount from about 2% to about 85% by weight of the dosage form. In an embodiment, non-swelling release retardant is present in the formulation in an amount from about 5% to about 80% by weight of the dosage form.

In one embodiment, the GABA analog in the form of, but not limited to, powder, granules, pellets, beads, minitablets or the like can be coated, blended or granulated with a non-swelling release retardant. In one embodiment, the GABA analog may be blended or physically mixed with the non-swelling release retardant. Granulation of the GABA analog with non-swelling release retardant can be done by any of the techniques known in the art such as, but not limited to, melt granulation, wet granulation, dry granulation or roll compaction. Coating of the GABA analog with non-swelling release retardant can be done by any of the techniques known in the art such as, but not limited to, fluid bed coating, pan coating, spray drying and the like. Coating can be carried out in the range from about 1 % to about 80% weight gain, preferably from about 2% to about 60%, more preferably from about 5 to about 50%. In one embodiment coated GABA analog is incorporated in the dosage forms of the present invention. Lipids or waxes can also be employed in the form of an aqueous dispersion stabilized by surfactants and suitable stabilizers for coating or granulating GABA analogs. In another embodiment, the GABA analog is coated with non-swelling release retardant by physically mixing the active with the release retardant.

The compositions of the present invention comprise in addition to at least one swelling agent and at least one non-swelling release retardant, pharmaceutically acceptable excipients such as, but not limited to, swelling enhancers, gas generating agent, binders, lubricants, diluents, disintegrants, glidants, colorants and the like.

Some excipients of a special category help the swelling agents to swell rapidly to a large extent resulting in a dramatic increase in the size of the tablet. At lower concentrations, these excipients are used as superdisintegrants; however at concentration above 5 % w/w these agents function as swelling enhancers and help increase the size of the dosage form. According to the present invention, suitable swelling enhancers include, but are not limited to, low-substituted hydroxypropyl cellulose, microcrystalline cellulose, cross-linked sodium or calcium carboxymethyl cellulose, cellulose fiber, cross-linked polyvinyl pyrrolidone, cross-linked polyacrylic acid, cross-linked Amberlite resin, alginates, colloidal magnesium-aluminum silicate, corn starch granules, rice starch granules, potato starch granules, pregelatinised starch, sodium starch glycolate, sodium carboxymethyl starch and the like or combinations thereof The content of the swelling enhancer in the compositions of the present invention is about 5% to about 90% by weight of the dosage form. In one embodiment, the content of the swelling enhancer is about 10% to about 70% by weight of the dosage form. In another embodiment, the content of the swelling enhancer is about 15% to about 50% by weight of the dosage form.

In one embodiment, the composition according to the present invention further comprises at least one swelling enhancer. When a combination of swelling agent and swelling enhancer is employed for gastric-retention, it allows a rapid and dramatic increase in the size of the tablets. Such a combination may be employed which allows rapid swelling and maintenance of integrity by polymeric network formed upon swelling of the polymer(s).

According to the present invention, the pharmaceutical composition can further comprise at least one gas generating agent. The gas generating agents also referred to as effervescent agent aid in the formation of highly porous, preferably honeycombed structure and enhances the buoyancy of the formulation. The gas generating agent employed for the purpose of the present invention is selected from, but not limited to, alkali and alkaline-earth metal carbonates and bicarbonates such as sodium bicarbonate, sodium glycine carbonate, potassium bicarbonate, ammonium bicarbonate, sodium bisulfite, sodium metabisulfite, sodium carbonate, potassium carbonate and the like. In one embodiment, the gas generating agent is sodium bicarbonate. The pharmaceutical composition can further optionally comprise an acid source. The acid source may be, but is not limited to, citric acid, maleic acid, oxalic acid, malonic acid, succinic acid, glutaric acid, adipic acid, fumaric acid, phthalic acid, aspartic acid, glutamic acid, malic acid or tartaric acid. In one embodiment, the gas generating agent is used at concentration from about 0.5 weight % to about 25 weight % of the dosage form. In another embodiment, the gas generating agent and the acid source are together used at a concentration from about 0.5 weight % to about 25 weight % of the dosage form.

The compositions of the present invention typically may also include other pharmaceutically acceptable excipients such as, but not limited to, binders, lubricants, diluents, disintegrants, glidants, colorants and the like. As is well known to those skilled in the art, pharmaceutical excipients are routinely incorporated into solid dosage forms. This is done to ease the manufacturing process as well as to improve the performance of the dosage form.

Examples of suitable binders include, but are not limited to, starch, pregelatinized starch, polyvinyl prrolidone (PVP), copovidone, cellulose derivatives, such as hydroxypropylmethyl cellulose (HPMC), hydroxypropyl cellulose (HPC) and carboxymethyl cellulose (CMC) and their salts. Examples of suitable diluents include, but are not limited to, starch, dicalcium phosphate, microcrystalline cellulose, lactose monohydrate, dextrate hydrated and the like. Examples of suitable lubricants include, but are not limited to, magnesium stearate, calcium stearate, stearic acid, talc, and sodium stearyl fumarate. Compositions of the present invention may optionally also include a glidant such as, but not limited to, colloidal silica, silica gel, precipitated silica, or combinations thereof. Suitable disintegrants employed in the compositions of the present invention include croscarmellose sodium, crospovidone, sodium starch glycolate, starch or combinations thereof.

The controlled release gastroretentive dosage forms as per the present invention may be in the form of a monolithic system, an expanding bilayered or multilayered or in-lay system for oral administration which is adapted to deliver the active agent in a modified manner over extended period. GABA analog may be incorporated in monolithic matrix type of controlled release gastroretentive dosage form. In one embodiment, the gastroretentive dosage form of the present invention is multi-layered. In another embodiment, the gastroretentive dosage form of the present invention is a bilayered dosage form comprising an active layer and a gastroretentive layer. In a further embodiment, GABA analog is incorporated in the drug layer of a bilayered controlled release gastroretentive dosage form that consists of a drug layer and a gastroretentive expanding layer wherein the drug is released in a sustained manner over a desired time period from the drug layer. In another embodiment, the bilayered gastroretentive dosage form comprises a) an active layer comprising GABA analog, at least one non-swelling pH-dependent polymeric release retardant, at least one pharmaceutically acceptable excipient and optionally at least one swelling agent; b) a gastroretentive layer comprising at least one swelling agent and at least one pharmaceutically acceptable excipient. In one embodiment, the bilayered gastroretentive dosage form comprises a) an active layer comprising GABA analog, at least one non-swelling pH dependent polymeric release retardant, at least one pharmaceutically acceptable excipient and optionally at least one swelling agent; b) a gastroretentive layer comprising at least one swelling agent, at least one swelling enhancer and at least one pharmaceutically acceptable excipient. In a further embodiment, the multilayered gastroretentive dosage form comprises a) an active layer comprising GABA analog, at least one non-swelling pH-dependent polymeric release retardant, at least one pharmaceutically acceptable excipient and optionally at least one swelling agent; b) one or more gastroretentive layer comprising at least one swelling agent and at least one pharmaceutically acceptable excipient. In case wherein the dosage form is a multilayered tablet with drug layer and gastroretentive layer/s the swelling agents ensure that there is sufficient swelling for retention of the dosage form despite erosion of the drug layer.

In a further embodiment a solid pharmaceutical composition in the form of an expanding bilayered system for oral administration is provided to deliver GABA analog from a first layer immediately upon reaching the gastrointestinal tract, and to deliver same or different analog, from a second layer, in a sustained manner over a specific time period. The second layer is also adapted to provide expanding nature for the dosage system, thereby making the dosage system have greater retention in the upper gastrointestinal tract. In yet another embodiment, the controlled release gastroretentive dosage form is in the form of a trilayered system consisting of a drug layer compressed between a first gastroretentive layer and a second gastroretentive layer wherein GABA analog is released in a sustained manner from the drug layer. In a further embodiment, the controlled release gastroretentive dosage form is in the form of a trilayered system consisting of an immediate release layer designed to deliver a GABA analog immediately upon reaching the gastrointestinal tract, a sustained release layer adapted to deliver the same or different GABA analog in a sustained manner over a desired time period and a gastroretentive layer designed to cause retention of the dosage form in the upper gastrointestinal tract. In a further embodiment the controlled release gastroretentive dosage form of the present invention comprises GABA analog blended, granulated or coated with a non-swelling release retardant. The solid pharmaceutical compositions of the present invention for oral administration of GABA analog ensure desired gastroretention and sustained delivery of the active.

In yet another embodiment, the controlled release gastroretentive dosage form is in the form of a trilayered system consisting of a drug layer compressed between a gastroretentive layer and a barrier layer wherein GABA analog is released in a sustained manner from the drug layer. The barrier layer acts as a barrier modulating the release and is partially impermeable, for a predeterminable time, to the active ingredient contained in the adjacent drug layer. In one embodiment the excipients employed for the preparation of said barrier layer include but are not limited to, glyceryl monostearate and derivative thereof, semisynthetic glycerides, hydrogenated castor oil, glyceryl palmitostearate, glyceryl behenate, cetyl alcohol, glycerin, cellulose derivatives, ethylcellulose, methylcellulose, sodium carboxymethylcellulose, polymethacrylates, polyvinylpyrrolidone, stearic acid, talc, sodium benzoate, boric acid, polyoxyethylene glycols, colloidal silica and the like. Further for the preparation of barrier layer, plasticizers may be employed that provide said barrier layer with the elasticity required and to improve their compressibility, such as but not limited to hydrogenated vegetable oils, fatty alcohols, fatty acids, glycerides and triglycerides and their substituted forms, polyoxyethylene glycols and derivatives thereof and the like. In one embodiment the barrier layer may also be characterized in that it can act as a barrier modulating the release and can rapidly swell, i.e. can rapidly increase in volume, and have bioadhesive properties allowing dosage form retention and adhesion to gastrointestinal mucosa.

In a further embodiment a solid pharmaceutical composition of the present invention is in the form of an in-lay system which a specialized dosage form comprising a drug containing tablet which is placed in another tablet comprising a blend of excipients that ensure gastric retention. In this system the drug containing tablet is small and is covered from all sides except at least one side with a blend of excipient that ensure the gastric retention.

In yet another illustrative embodiment according to the invention, the dosage form may be optionally coated. Surface coatings may be employed for aesthetic purposes or for dimensionally stabilizing the compressed dosage form or for retarding the drug release. The surface coating may be any conventional coating which is suitable for enteral use. The coating may be carried out using any conventional technique employing conventional ingredient. A surface coating can, for example, be obtained using a quick-dissolving film using conventional polymers such as, but not limited to, hydroxypropyl methyl cellulose, hydroxypropyl cellulose, carboxymethyl cellulose, ethyl cellulose, polyvinyl alcohol, poly methacrylates or the like.

Tablets of the present invention may vary in shape including, but not limited to, oval, triangle, almond, peanut, parallelogram, pentagonal. It is contemplated within the scope of the invention that the dosage form can be encapsulated. Tablets in accordance with the present invention may be manufactured using conventional techniques of common tableting methods known in the art such as direct compression, wet granulation, dry granulation and extrusion/ melt granulation.

The present disclosure provides a method of preparing a controlled release gastroretentive dosage form of GABA analog for once a day or twice a day administration comprising: (a) coating a GABA analog with at least one non-swelling release retardant to form a coated GABA analog; (b) mixing said coated GABA analog with at least one pharmaceutically acceptable excipient and lubricant to form a drug layer; (c) blending at least one swelling agent, at least one pharmaceutically acceptable excipient and lubricant to form a gastroretentive layer; and (d) compressing the drug layer and the gastroretentive layer to form a bilayer tablet. The present disclosure also provides a method of preparing a controlled release gastroretentive dosage form of GABA analog for once a day or twice a day administration comprising: (a) blending a GABA analog with at least one non-swelling release retardant and at least one pharmaceutically acceptable excipient and lubricant to form a drug layer blend;(b) blending at least one swelling agent, at least one pharmaceutically acceptable excipient and lubricant to form a gastroretentive layer; and (c) compressing the drug layer and the gastroretentive layer to form a bilayer tablet.

The present disclosure provides a method of preparing a controlled release gastroretentive dosage form of GABA analog for once a day or twice a day administration comprising (a) granulating a GABA analog with at least one non-swelling release retardant and at least one pharmaceutically acceptable excipient to form drug granules; (b) blending the drug granules with remaining pharmaceutically acceptable excipients to form drug layer; (c) blending at least one swelling agent, at least one pharmaceutically acceptable excipient and lubricant to form a gastroretentive layer; and (d) compressing the drug layer and the gastroretentive layer to form a bilayer tablet. In another embodiment, the present disclosure provides a method of preparing a controlled release gastroretentive dosage form of GABA analog for once a day or twice a day administration comprising: (a) blending a GABA analog with at least one non-swelling release retardant and at least one pharmaceutically acceptable excipient and lubricant to form a drug layer blend; (b) granulating at least one swelling agent, at least one pharmaceutically acceptable excipient to form granules; (c) blending the granules of step (b) with remaining swelling agents and pharmaceutically acceptable excipients and lubricant to form a gastroretentive layer; and (d) compressing the drug layer and the gastroretentive layer to form a bilayer tablet.

The dosage form having a single layer or multi-layer composition, coated or uncoated, swells after ingestion gradually upon contact with gastric fluid. The time taken for swelling may vary from about 15 minutes to about 4 hours. In one embodiment, the time taken for swelling is within about 15 minutes to about 3 hours. In another embodiment, the time taken for swelling is within about 15 minutes to about 2 hours. Two dimensions of the dosage form e.g. length and width expand to more than about 8 mm within about 4 hours. In one embodiment, two dimensions of the dosage form e.g. length and width of the dosage form expand to more than about 10 mm within about 4 hours. In another embodiment, two dimensions of the dosage form e.g. the length and width of the dosage form expand to more than about 12 mm within about 4 hours. In one embodiment from a bilayered tablet formulation, GABA analog is released by an erosion mechanism. This means that, to achieve a desired release profile, drug layer has to continuously erode or, in other words, the size of the drug layer decreases continuously. Thus there are two antagonistic phenomena occurring simultaneously in such formulations, namely, one - swelling of the dosage form for gastric retention and two - erosion of the drug layer. It was surprisingly found that despite of erosion of the drug layer, gastric retention can be achieved. The pyloric sphincter, controlling the movement of the contents of the stomach into the intestine is 8-10 mm in diameter. Gastric retention is thus possible by ensuring that at least two dimensions of the dosage form are greater than 8 mm after swelling within 2 hours in media simulating typical gastric environment (0.1N hydrochloric acid).

In one embodiment following oral administration the dosage form is retained in the upper gastrointestinal tract for a time period of about 30 min to about 12 hours. In one embodiment, the dosage form is retained in the upper gastrointestinal tract for a time period of about 1 hour to about 10 hours. In another embodiment, the dosage form is retained in the upper gastrointestinal tract for a time period of about 1 hour to about 8 hours.

The controlled release gastroretentive dosage forms formulated according to the present invention allow for controlled release of GABA analog. In one embodiment the in vitro release rate corresponds to the following % rate of GABA analog released as shown in the following tables which is determined in USP dissolution apparatus II (rotation speed of 50 rpm) using a suitable buffer preferably 0.1N hydrochloric acid.

**TABLE A**

| TIME (H) | % RELEASED |
|---|---|
| 1 | 10-40 |
| 2 | 20-55 |
| 4 | 35-70 |
| 6 | 50-90 |
| 8 | 60-95 |
| 12 | 70-100 |

In another embodiment, the formulation particularly suited for once-a-day dosing has an in-vitro release rate corresponding to the following % rate of GABA analog released as shown in Table B determined in USP dissolution apparatus II (rotation speed of 50 rpm) using a suitable buffer preferably 0.1N hydrochloric acid.

**TABLE B**

| TIME (H) | % RELEASED |
|---|---|
| 1 | 5-45 |
| 2 | 15-55 |
| 4 | 30-75 |
| 8 | 45-90 |
| 12 | 70-95 |
| 16 | 80-100 |
| 24 | >85 |

Further, the present invention is also applicable to combinations of GABA analogs with other drugs. In one embodiment, compositions of the present invention comprise GABA analog in combination with other active agents selected from, but not limited to, celecoxib, diclofenac, diflunisal, flurbiprofen, naproxen, nimesulide, sulindac, pramipexole, donepezil, oxybutynin, sildenafil, vardenafil, tadalafil, caffeine, meloxicam, amantadine, memantine, riluzole and tramadol.

In a further embodiment is provided the use of gastroretentive dosage forms of GABA analog of the present invention for the manufacture of a medicament for the treatment of epilepsy, faintness attacks, hypokinesia, cranial traumas, neurodegenerative disorders such as Alzheimer's disease, Huntington's chorea or Parkinson's disease and amyotrophic lateral sclerosis, depression, mania and bipolar disorders, anxiety, panic inflammation, renal colic, insomnia, gastrointestinal damage, incontinence, pain including neuropathic pain, muscular pain, skeletal pain and migraine. Further, the present invention provides a method of treating epilepsy, faintness attacks, hypokinesia, cranial traumas, neurodegenerative disorders such as Alzheimer's disease, Huntington's chorea or Parkinson's disease and amyotrophic lateral sclerosis, depression, mania and bipolar disorders, anxiety, panic inflammation, renal colic, insomnia, gastrointestinal damage, incontinence, pain including neuropathic pain, muscular pain, skeletal pain and migraine, comprising administering to the subject in need thereof gastroretentive dosage forms of the present invention.

While the present invention has been described in terms of its specific illustrative embodiments, certain modifications and equivalents will be apparent to those skilled in the art . The details of the invention, its objects and advantages are explained hereunder in greater detail in relation to nonlimiting exemplary illustrations.

### Examples

### Comparative Example 1: Gastroretentive tablet of pregabalin

### A. Preparation of drug layer

**Table 1: Composition of drug granules**

| **Ingredients** | **mg/tablet** |
|---|---|
| Pregabalin | 150 |
| Glyceryl behenate, USP | 18 |
| Mannitol, USP | 45 |

Procedure: Glyceryl behenate was melted and to the molten mass were added pregabalin and mannitol and allowed to solidify to form granules.

**Table 2: Composition of sustained release drug layer**

| **Ingredients** | **mg/tablet** |
|---|---|
| Pregabalin granules | 213 |
| Ethyl cellulose, USP | 80 |
| Microcrystalline cellulose, USP | 30 |
| Povidone, USP | 10 |
| Sodium starch glycolate, USP | 20 |
| Magnesium stearate, USP | 5 |
| **Weight of drug layer** | **355** |

Procedure: The granules obtained as per the composition of Table 1 were blended with other excipients except lubricant, and then lubricated to produce the drug layer.

### B. Preparation of gastroretentive layer

**Table 3: Composition of gastroretentive layer**

| **Ingredients** | **mg/tablet** |
|---|---|
| Polyethylene oxide, USP | 80 |
| Hydroxy propyl methyl cellulose, USP | 125 |
| Hydroxy ethyl cellulose, USP | 75 |
| Crospovidone, USP | 80 |
| Povidone, USP | 15 |
| Microcrystalline cellulose, USP | 35 |
| Sodium bicarbonate, USP | 20 |
| Magnesium stearate, USP | 5 |
| **Weight of gastroretentive layer** | **435** |

Procedure: All ingredients except lubricant, binder and gas-generating agent were dry mixed in rapid mixer granulator, granulated using povidone solution and subsequently dried in a fluidized bed dryer to get desired loss on drying. Sized dried granules were blended with sodium bicarbonate and then lubricated using magnesium stearate to form the gastroretentive layer.

A bilayer gastroretentive tablet of pregablin was prepared by compressing the drug layer and the gastroretentive layer.

### Comparative Example 2: Gastroretentive tablet of pregabalin

Composition of drug granules: 150mg pregabalin was coated with ammonio methacrylate copolymer, type B to a weight gain of up to 30% to obtain coated granules.

**Table 4: Composition of gastroretentive tablet of pregabalin**

| **Ingredients** | **mg/tablet** |
|---|---|
| Pregabalin coated granules | 195 |
| Hydroxy propyl methyl cellulose, USP | 315 |
| Carbomer 934P, USP | 80 |
| Crospovidone, USP | 65 |
| Povidone, USP | 20 |
| Microcrystalline cellulose, USP | 35 |
| Sodium bicarbonate, USP | 22 |
| Citric acid, USP | 11 |
| Magnesium stearate, USP | 7 |
| **Total** | **750** |

Procedure: The coated granules of pregabalin were mixed with other excipients and the blend was lubricated and compressed to form gastroretentive tablet.

Swelling study of the compressed tablet in 0.1N HCl:
Swelling of the tablet *in-vitro* is determined in USP Type II dissolution apparatus at 75 rpm using 900ml 0.1N hydrochloric acid dissolution media and at temperature of 37 ± 0.5°C; with dimensions measured physically using vernier calipers at the end of 1 hour. Width of the tablet is noted.

| | |
|---|---|
| Tablet dimension (mm) | 22 X 10 |
| Width in 1 hr. in 0.1N HCl (mm) | 12.23 |

### In vitro dissolution study

*In-vitro* dissolution studies of gastroretentive tablets of pregabalin were carried out in 900 ml 0.1N HCl with following specifications:
Dissolution Test Apparatus: USP Type II; Temperature: 37 ± 0.5°C; Dissolution Medium: 900 ml 0.1N HCl; rpm: 50

**Table 5: Dissolution data**

| **Time (hr)** | **% Drug Release** |
|---|---|
| 1 | 28.2 |
| 2 | 40.5 |
| 4 | 53.7 |
| 6 | 62.4 |
| 8 | 75.8 |
| 12 | 95.1 |

### Comparative Example 3: Gastroretentive tablet of pregabalin

A multilayer gastroretentive tablet of pregabalin was prepared comprising a gastroretentive layer, a sustained release drug layer and an immediate release drug layer.

### A. Gastroretentive layer

**Table 6: Composition of gastroretentive layer**

| **Ingredients** | **mg/tablet** |
|---|---|
| Hydroxy propyl methyl cellulose, USP | 250 |
| Xanthan gum, USP | 50 |
| Crospovidone, USP | 65 |
| Povidone, USP | 20 |
| Microcrystalline cellulose, USP | 15 |
| Sodium bicarbonate, USP | 15 |
| Magnesium stearate, USP | 5 |
| **Weight of gastroretentive layer** | **420** |

Procedure: The gastroretentive layer was prepared as per procedure described under example 1.

### B. Sustained release drug layer

i) Composition of drug granules: 100mg pregabalin was coated with cellulose acetate phthalate to up to 20% weight gain to obtain coated pregabalin granules.
ii) Preparation of sustained release drug layer

**Table 7: Composition of sustained release drug layer**

| **Ingredients** | **mg/tablet** |
|---|---|
| Pregabalin coated granules | 120 |
| Hydroxy propyl methyl cellulose, USP | 75 |
| Microcrystalline cellulose, USP | 20 |
| Povidone, USP | 18 |
| Sodium starch glycolate, USP | 25 |
| Magnesium stearate, USP | 7 |
| **Weight of drug layer** | **265** |

Procedure: The coated granules of pregabalin were blended with all excipients, except lubricant then lubricated to form the sustained release drug layer.

### C. Immediate release drug layer

**Table 8: Composition of immediate release drug layer**

| **Ingredients** | **mg/tablet** |
|---|---|
| Pregabalin | 50 |
| Microcrystalline cellulose, USP | 55 |
| Povidone, USP | 5 |
| Sodium starch glycolate, USP | 8 |
| Magnesium stearate, USP | 2 |
| **Weight of drug layer** | **120** |

Procedure: The immediate release drug layer was prepared by blending all the excipients except lubricant and then lubricating with magnesium stearate.

The gastroretentive layer, sustained release drug layer and immediate release drug layer were compressed together to form a multilayer gastroretentive tablet of pregabalin.

### Comparative Example 4: Gastroretentive tablet of gabapentin

### A. Preparation of drug layer

**Table 9: Composition of drug granules**

| **Ingredients** | **mg/tablet** |
|---|---|
| Gabapentin | 300 |
| Hydrogenated vegetable oil, USPNF | 150 |

Procedure: Hydrogenated vegetable oil was melted and gabapentin was added to the molten mass and allowed to solidify to form granules.

**Table 10: Composition of sustained release drug layer**

| **Ingredients** | **mg/tablet** |
|---|---|
| Gabapentin granules | 450 |
| Microcrystalline cellulose, USP | 105 |
| Povidone, USP | 12 |
| Sodium starch glycolate, USP | 25 |
| Magnesium stearate, USP | 8 |
| **Weight of drug layer** | **600** |

Procedure: The drug layer was prepared by blending gabapentin granules with all excipients except lubricant followed by lubrication with magnesium stearate to form drug layer.

### B. Preparation of gastroretentive layer

**Table 11: Composition of gastroretentive layer**

| **Ingredients** | **mg/tablet** |
|---|---|
| Polyethylene oxide, USP | 125 |
| Hydroxy propyl methyl cellulose, USP | 160 |
| Hydroxy ethyl cellulose, USP | 100 |
| Crospovidone, USP | 85 |
| Povidone, USP | 25 |
| Microcrystalline cellulose, USP | 50 |
| Magnesium stearate, USP | 5 |
| **Weight of gastroretentive layer** | **550** |

Procedure: All ingredients except lubricant and binder were dry mixed in rapid mixer granulator, granulated using povidone solution and subsequently dried in a fluidized bed dryer to get desired loss on drying. Sized dried granules were then lubricated using magnesium stearate.

A bilayer gastroretentive tablet of gabapentin was prepared by compressing the drug layer and the gastroretentive layer.

### Comparative Example 5: Gastroretentive tablet of gabapentin

### A. Preparation of drug layer

i) Composition of drug granules: 300mg gabapentin was coated with ethylcellulose to up to 25% weight gain to obtain gabapentin granules.
ii) The drug layer was prepared as per the following composition

**Table 12: Composition of sustained release drug layer**

| **Ingredients** | **mg/tablet** |
|---|---|
| Gabapentin coated granules | 375 |
| Microcrystalline cellulose, USP | 80 |
| Povidone, USP | 15 |
| Sodium starch glycolate, USP | 25 |
| Magnesium stearate, USP | 5 |
| **Weight of drug layer** | **500** |

### B. Preparation of gastroretentive layer

**Table 13: Composition of gastroretentive layer**

| **Ingredients** | **mg/tablet** |
|---|---|
| Hydroxy propyl methyl cellulose, USP | 375 |
| Xanthan gum, USP | 120 |
| Crospovidone, USP | 85 |
| Povidone, USP | 35 |
| Microcrystalline cellulose, USP | 50 |
| Sodium bicarbonate, USP | 27 |
| Magnesium stearate, USP | 8 |
| **Weight of gastroretentive layer** | **700** |

Procedure: Lubricated blend of drug layer and gastroretentive layer was prepared as per procedure described under example 1 and the tablets were compressed to obtain bilayer gastroretentive tablet of gabapentin.

### Comparative Example 6: Gastroretentive tablet of gabapentin

A gastroretentive tablet of gabapentin was prepared comprising a drug layer present in between an upper gastroretentive layer and a lower gastroretentive layer.

### A. Preparation of upper gastroretentive layer

**Table 14: Composition of upper gastroretentive layer**

| **Ingredients** | **mg/tablet** |
|---|---|
| Polyethylene oxide, USP | 40 |
| Hydroxy propyl methyl cellulose, USP | 100 |
| Hydroxy ethyl cellulose, USP | 25 |
| Crospovidone, USP | 30 |
| Povidone, USP | 8 |
| Microcrystalline cellulose, USP | 15 |
| Sodium bicarbonate, USP | 10 |
| Magnesium stearate, USP | 2 |
| **Weight of gastroretentive layer** | **230** |

Procedure: All ingredients were dry mixed in rapid mixer granulator, granulated using povidone solution and subsequently dried in a fluidized bed dryer to get desired loss on drying. Sized dried granules were blended with sodium bicarbonate and then lubricated using magnesium stearate to give the gastroretentive layer.

### B. Preparation of drug layer

**Table 15: Composition of sustained release drug layer**

| **Ingredients** | **mg/tablet** |
|---|---|
| Gabapentin | 300 |
| Ammonio methacrylate copolymer, Type A, USPNF | 100 |
| Hydroxy propyl methyl cellulose, USP | 150 |
| Microcrystalline cellulose, USP | 50 |
| Povidone, USP | 15 |
| Crospovidone, USP | 30 |
| Magnesium stearate, USP | 5 |
| **Weight of drug layer** | **650** |

Procedure: The drug layer was prepared by blending gabapentin with ammmonio methacrylate copolymer, type A, followed by mixing with other excipients and lubricated to form a uniform blend.

### C. Preparation of lower gastroretentive layer

**Table 16: Composition of lower gastroretentive layer**

| **Ingredients** | **mg/tablet** |
|---|---|
| Polyethylene oxide, USP | 40 |
| Hydroxy propyl methyl cellulose, USP | 100 |
| Hydroxy ethyl cellulose, USP | 30 |
| Crospovidone, USP | 30 |
| Povidone, USP | 5 |
| Microcrystalline cellulose, USP | 15 |
| Sodium bicarbonate, USP | 8 |
| Magnesium stearate, USP | 2 |
| **Weight of gastroretentive layer** | **230** |

Procedure: The lower gastroretentive layer was prepared in the same manner as described for upper gastroretentive layer.

The lubricated blends obtained for the three layers were compressed to form trilayered gastroretentive tablet of gabapentin.

### Example 7: Gastroretentive tablet of pregabalin

A gastroretentive tablet of pregabalin was prepared comprising an active layer and a drug layer.

### A. Preparation of drug layer

**Table 17: Composition of active drug layer**

| **Ingredients** | **mg/tablet** |
|---|---|
| Pregablin | 330 |
| Hydroxypropyl methylcellulose, USP | 130 |
| Methacrylic Acid - ethyl acrylate copolymer (1:1)Ph. Eur/NF | 30 |
| Copovidone, Ph.Eur | 30 |
| Microcrystalline cellulose, NF, Ph.Eur. | 95 |
| Iron oxide yellow | 1 |
| Magnesium stearate | 10 |
| Talc | 4 |
| **Total** | 630 |

Procedure: Pregabalin, hydroxypropyl methylcellulose and methacrylic acid-ethyl acrylate copolymer (1:1) were blended. This blend was blended with all the remaining excipients except magnesium stearate. The final blend is then lubricated with magnesium stearate to form drug layer.

### B. Preparation of gastroretentive layer

**Table 18: Composition of gastroretentive layer**

| **Ingredients** | **mg/tablet** |
|---|---|
| Polyethylene oxide | 130 |
| Hydroxypropyl methylcellulose, USP | 130 |
| Hydroxy ethyl cellulose, | 65 |
| Crospovidone, USP/NF | 138 |
| Copovidone, Ph.Eur. | 63 |
| Microcrystalline cellulose, NF, Ph.Eur. | 110 |
| Sodium bicarbonate | 42 |
| Citric acid | 15 |
| Mg sterate | 4 |
| Colloidal silicon dioxide, USP | 3 |
| **Total** | 700 |
| **Weight of tablet** | 1330 |

Procedure: All the excipients except the lubricant were blended. The blend was lubricated with magnesium stearate to form gastroretentive layer.

A bilayer gastroretentive tablet of pregabalin was prepared by compressing the drug layer and the gastroretentive layer.

Swelling study of the compressed tablet:
Swelling of the tablet *in-vitro* is determined in USP Type II dissolution apparatus at 75 rpm using 900ml 0.1N hydrochloric acid dissolution media and at temperature of 37 ± 0.5°C; with dimensions measured physically using vernier calipers at the end of 1 hour. Width of the tablet is noted.

| | |
|---|---|
| Tablet dimension (mm) | 22 X 10 |
| Width in 1 hr. in 0.1N HCl (mm) | 12.1 |

### In vitro dissolution study

*In-vitro* dissolution studies of gastroretentive tablets of pregabalin were carried out in 900 ml 0.1N HCl with following specifications:
Dissolution Test Apparatus: USP Type II; Temperature: 37 ± 0.5°C; Dissolution Medium: 900 ml 0.1N hydrochloric acid; rpm: 50

**Table 19: Dissolution data**

| **Time (hr)** | **% Drug Release** |
|---|---|
| 1 | 29.6 |
| 2 | 44.5 |
| 4 | 62.7 |
| 6 | 79.8 |
| 8 | 89 |

## Claims

1. A gastroretentive dosage form comprising a GABA analog, at least one swelling agent, at least one non-swelling pH dependent polymeric release retardant and at least one pharmaceutically acceptable excipient;
wherein the GABA analog is selected from pregabalin, gabapentin, baclofen, vigabatrin, ((1 R,5S)-3-Amlnomethyl-1,5-dimethyl-bicyclo[3.2.0]hept-3-yl)-acetic acid; ((1 S,5R)-3-Aminomethyl-1,5-dimethyl-bicyclo[3.2.0]hept-3-yl)-acetic acid; ((1R,5S)-3-Aminomethyl-6,6-dimethyl-bicyclo[3.1.0]hex-3-yl)-acetic acid; ((1S,5R)-3-Aminomethyl-6,6-dimethyl-bicyclo[3.1.0]hex-3-yl)-acetic acid; ((1S,2S,5R)-2-Aminomethyl-6,6-dimethyl-bicyclo[3.1.0]hex-2-yl)-acetic acid; ((1 R,2S,5S)-2-Aminomethyl-6,6-dimethyl-bicyclo[3.1.0]hex-2-yl)-acetic acid; ((1S,2R,5R)-2-Aminomethyl-6,6-dimethyl-bicyclo[3.1.0]hex-2-yl)-acetic acid; ((1 R,2R,5S)-2-Aminomethyl-6,6-dimethyl-bicyclo[3.1.0]hex-2-yl)-acetic acid; ((1 R,5R,6S)-6-Aminomethyl-bicyclo[3.2.0]hept-6-yl)-acetic acid; ((1S,5S,6S)-6-Aminomethyl-bicyclo[3.2.0]hept-6-yl)-acetic acid; ((1 R,5R,6R)-6-Aminomethyl-bicyclo[3.2.0]hept- 6-yl)-acetic acid; ((1S,5S,6R)-6-Aminomethyl-bicyclo[3.2.0]hept-6-yl)-acetic acid; cis-((1S,2R,4S,5R)-3-Aminomethyl-2,4-dimethyl-bicyclo[3.2.0]hept-3-yl)-acetic acid; trans-((18,2R,48,5R)-3-Aminomethyl-2,4-dimethyl-bicyclo[3.2.0]hept-3-yl)-acetic acid; ((1S,5R,6S,7R)-3-Aminomethyl-6,7-dimethyl-bicyclo[3.2.0]hept-3-yl)-acetic acid; ((1S,5R,6R,7S)-3-Aminomethyl-6,7-dimethyl-bicyclo[3.2.0]hept-3-yl)-acetic acid; ((1R,2S,5S)-7-Aminomethyl-3,3-dimethyl-tricyclo[3.3.0.0]oct-7-yl)-acetic acid; ((1 R,6R,7S)-7-Aminomethyl-bicyclo[4.2.0]oct-7-yl)-acetic acid; ((1S,6S,7S)-7-Aminomethyl-bicyclo[4.2.0]oct-7-yl)-acetic acid; ((1 R,6R,7R)-7-Aminomethyl-bicyclo[4.2.0]oct-7-yl)-acetic acid; ((1S,6S,7R)-7-Aminomethyl-bicyclo[4.2.0]oct-7-yl)-acetic acid; ((1R,7R,8S)-8-Aminomethyl-bicyclo[5.2.0]non-8-yl)-acetic acid; ((1S,7S,8S)-8-Aminomethyl-bicyclo[5.2.0]non-8-yl)-acetic acid; ((1 R,7R,8R)-8-Aminomethyl-bicyclo[5.2.0]non-8-yl)-acetic acid; ((1S,7S,8R)-8-Aminomethyl-bicyclo[5.2.0]non-8-yl)-acetic acid. [(1 R,5R,6S)-6-(Aminomethyl)bicyclo[3.2.0]hept-6-yl]acetic acid; [(1S,5S,6R)-6-(Aminomethyl)bicyclo[3.2.0]hept-6-yl]acetic acid; (1RS,5RS,6RS)-6-(Aminomethyl)bicyclo[3.2.0]hept-6-yl]acetic acid; and [(1RS,6RS,7SR)-7-(Aminomethyl)bicyclo[4.2.0]oct-7-yl]acetic acid or a combination thereof;
wherein the amount of the swelling agent in the dosage form is from 5% to 70% by weight of the dosage form, and wherein the swelling agent(s) swell voluminously in the presence of gastric contents to increase the dosage form size such that its passage through the pyrolus is precluded; and
wherein the non-swelling pH dependent polymeric release retardant is present in the formulation in an amount from 2% to 85% by weight of the dosage form and wherein the non-swelling pH dependent polymeric release retardant is polymethacrylic acid polymer, or a derivative thereof selected from methyl acrylate' acrylic acid copolymer, methyl acrylate'methacrylic acid copolymer, methacrylic acid'methyl methacrylate copolymer, methacrylic acid'ethyl acrylate copolymer, methyl acrylate methacrylic acid octyl acrylate copolymer or a combination thereof.

2. The dosage form of claim 1, wherein the GABA analog is pregabalin, gabapentin, baclofen or vigabatrin.

3. The dosage form of claim 1, wherein the swelling agent is polyalkylene oxide, cellulosic polymer, acrylic acid polymer, maleic anhydride polymer; polymaleic acid, poly(acrylamide), poly(olefinic alcohol), poly(N-vinyl lactam), polyol, polyoxyethylated saccharide, polyoxazoline, polyvinylamine, polyvinyl alcohol, polyimine, polysaccharide, polyurethane hydrogel, zein, shellac-based polymer, polyethylene oxide, hydroxypropyl cellulose, hydroxypropyl methyl cellulose, hydroxyethyl cellulose, sodium carboxymethyl cellulose, calcium carboxymethyl cellulose, methyl cellulose, polyacrylic acid, xanthan gum, and polyvinyl alcohol or derivative, or mixture thereof.

4. The dosage form of claim 1, wherein the pharmaceutically acceptable excipient is a swelling enhancer, a gas generating agent, a binder, a lubricant, a diluent, a disintegrant, a glidant, or a colorant.

5. The dosage form of claim 1, wherein the dosage form is in the form of bilayered or multilayered or in-lay system.

6. The dosage form of claim 5, wherein the dosage form is in the form of a bilayered gastroretentive dosage form.

7. The dosage form of claim 6, wherein the bilayered gastroretentive dosage form comprises (a) active layer and (b) gastroretentive layer.

8. The dosage form of claim 7, wherein the active layer comprises GABA analog, at least one non-swelling pH dependent polymeric release retardant polymethacrylic acid polymer or derivative thereof, at least one pharmaceutically acceptable excipient, and optionally at least one swelling agent; and the gastroretentive layer comprises at least one swelling agent and at least one pharmaceutically acceptable excipient.

9. The dosage form of claim 1, wherein the dosage form is retained in the upper gastrointestinal tract for a time period of about 30 min to about 12 hours.

10. The dosage form of claim 1, wherein the dosage form releases the GABA analog over a period of up to about 24 hours.

## Patentansprüche

1. Gastroretentive Darreichungsform, umfassend ein GABA-Analog, mindestens ein Quellmittel, mindestens ein nicht quellendes, pH-Wert-abhängiges polymeres Freisetzungshemmmittel und mindestens einen pharmazeutisch akzeptablen Träger;
wobei das GABA-Analog ausgewählt ist aus Pregabalin, Gabapentin, Baclofen, Vigabatrin, ((1R,5S)-3-Aminomethyl-1,5-dimethyl-bicyclo[3.2.0]hept-3-yl)-Essigsäure; ((1S,5R)-3-Aminomethyl-1,5-dimethyl-bicyclo[3.2.0]hept-3-yl)-Essigsäure; ((1R,5S)-3-Aminomethyl-6,6-dimethyl-bicyclo[3.1.0]hex-3-yl)-Essigsäure; ((1S,5R)-3-Aminomethyl-6,6-dimethyl-bicyclo[3.1.0]hex-3-yl)-Essigsäure; ((1S,2S,5R)-2-Aminomethyl-6,6-dimethyl-bicyclo[3.1.0]hex-2-yl)-Essigsäure; ((1 R,2S,5S)-2-Aminomethyl-6,6-dimethyl-bicyclo[3.1.0]hex-2-yl)-Essigsäure; ((1S,2R,5R)-2-Aminomethyl-6,6-dimethyl-bicyclo[3.1.0]hex-2-yl)-Essigsäure; ((1 R,2R,5S)-2-Aminomethyl-6,6-dimethyl-bicyclo[3.1.0]hex-2-yl)-Essigsäure; ((1 R,5R,6S)-6-Aminomethyl-bicyclo[3.2.0]hept-6-yl)-Essigsäure; ((1S,5S,6S)-6-Aminomethyl-bicyclo[3.2.0]hept-6-yl)-Essigsäure; ((1 R,5R,6R)-6-Aminomethyl-bicyclo[3.2.0]hept-6-yl)-Essigsäure; ((1 S,5S,6R)-6-Aminomethyl-bicyclo[3.2.0]hept-6-yl)-Essigsäure; cis-((1S,2R,4S,5R)-3-Aminomethyl-2,4-dimethyl-bicyclo[3.2.0]hept-3-yl)-Essigsäure; trans-((1S,2R,4S,5R)-3-Aminomethyl-2,4-dimethyl-bicyclo[3.2.0]hept-3-yl)-Essigsäure; ((1S,5R,6S,7R)-3-Aminomethyl-6,7-dimethyl-bicyclo[3.2.0]hept-3-yl)-Essigsäure; ((1S,5R,6R,7S)-3-Aminomethyl-6,7-dimethyl-bicyclo[3.2.0]hept-3-yl)-Essigsäure; ((1R,2S,5S)-7-Aminomethyl-3,3-dimethyl-tricyclo[3.3.0.0]oct-7-yl)-Essigsäure; ((1R,6R,7S)-7-Aminomethyl-bicyclo[4.2.0]oct-7-yl)-Essigsäure; ((1S,6S,7S)-7-Aminomethyl-bicyclo[4.2.0]oct-7-yl)-Essigsäure; ((1 R,6R,7R)-7-Aminomethyl-bicyclo[4.2.0]oct-7-yl)-Essigsäure; ((1S,6S,7R)-7-Aminomethyl-bicyclo[4.2.0]oct-7-yl)-Essigsäure; ((1R,7R,8S)-8-Aminomethyl-bicyclo[5.2.0]non-8-yl)-Essigsäure; ((1S,7S,8S)-8-Aminomethyl-bicyclo[5.2.0]non-8-yl)-Essigsäure; ((1R,7R,8R)-8-Aminomethyl-bicyclo[5.2.0]non-8-yl)-Essigsäure; ((1S,7S,8R)-8-Aminomethyl-bicyclo[5.2.0]non-8-yl)-Essigsäure; ((1R,5R,6S)-6-(Aminomethyl)bicyclo[3.2.0]hept-6-yl)-Essigsäure; ((1S,5S,6R)-6-(Aminomethyl)bicyclo[3.2.0]hept-6-yl)-Essigsäure; ((1RS,5RS,6RS)-6-(Aminomethyl)bicyclo[3.2.0]hept-6-yl)-Essigsäure; und ((1 RS,6RS,7SR)-7-(Aminomethyl)bicyclo[4.2.0]oct-7-yl)-Essigsäure; oder einer Kombination daraus;
wobei die Menge des Quellmittels in der Darreichungsform zwischen 5 und 70 Gew.-% der Darreichungsform entspricht, und wobei das Volumen des/der Quellmittel in Gegenwart von Mageninhalt aufquellt, um die Größe der Darreichungsform so zu erhöhen, dass deren Verlauf durch den Pyrolus verhindert wird; und
wobei das ein nicht quellende, pH-Wert-abhängige polymere Freisetzungshemmmittel in der Formulierung in einer Menge von 2 bis 85 Gew.-% der Darreichungsform vorhanden ist, und wobei das nicht quellende, pH-Wert-abhängige polymere Freisetzungshemmmittel Polymethacrylsäure-Copolymer oder ein Derivat davon ist, ausgewählt aus Methylacrylatacrylsäure-Copolymer, Methylacrylatmethacrylsäure-Copolymer, Methacrylsäuremethylmethacrylat-Copolymer, Methacrylsäureethylacrylat-Copolymer, Methylacrylatmethacrylsäureoctylacrylat-Copolymer oder einer Kombination davon.

2. Darreichungsform nach Anspruch 1, wobei das GABA-Analog Pregabalin, Gabapentin, Baclofen oder Vigabatrin ist.

3. Darreichungsform nach Anspruch 1, wobei das Quellmittel Polyalkylenoxid, Cellulosepolymer, Acrylsäurepolymer, Maleinanhydridpolymer, Polymaleinsäure, Poly(acrylamid), Poly(olefinalkohol), Poly/N-Vinyllactam), Polyol, polyoxyethyliertes Saccharid, Polyoxazolin, Polyvinylamin, Polyvinylalkohol, Polyimin, Polysaccharaid, Polyurethanhydrogelzein, Polymer auf Schellackbasis, Polyethylenoxid, Hydroxypropylcellulose, Hydroxypropylmethylcellulose, Hydroxyethylcellulose, Natriumcarboxymethylcellulosse, Calciumcarboxymethylcellulose, Methylcellulose, Polyacrylsäure, Xanthangummi und Polyvinylalkohol oder ein Derivat oder ein Gemisch davon ist.

4. Darreichungsform nach Anspruch 1, wobei der pharmazeutisch akzeptable Träger ein Quellungsfördermittel, ein Gaserzeugungsmittel, ein Bindemittel, ein Gleitmittel, ein Verdünner, ein Sprengmittel, ein Fließregulierungsmittel oder ein Farbstoff ist.

5. Darreichungsform nach Anspruch 1, wobei die Darreichungsform in Form eines zweilagigen oder mehrlagigen oder Inlay-Systems gegeben ist.

6. Darreichungsform nach Anspruch 5, wobei die Darreichungsform in Form einer zweilagigen gastroretentiven Darreichungsform gegeben ist.

7. Darreichungsform nach Anspruch 6, wobei die zweilagige gastroretentive Darreichungsform (a) eine aktive Lage und (b) eine gastroretentive Lage umfasst.

8. Darreichungsform nach Anspruch 7, wobei die aktive Lage ein GABA-Analog, mindestens ein nicht quellendes, pH-Wert-abhängiges polymeres Freisetzungshemmmittel, ein Polymethacrylsäurepolymer oder Derivat davon, mindestens einen pharmazeutisch akzeptablen Träger und optional mindestens ein Quellmittel umfasst; und wobei die gastroretentive Lage mindestens ein Quellmittel und mindestens einen pharmazeutisch akzeptablen Träger umfasst.

9. Darreichungsform nach Anspruch 1, wobei die Darreichungsform über einen Zeitraum zwischen etwa 30 Minuten und etwa 12 Stunden im oberen Magen-DarmTrakt verbleibt.

10. Darreichungsform nach Anspruch 1, wobei die Darreichungsform das GABA-Analog über einen Zeitraum von bis zu 24 Stunden freisetzt.

## Revendications

1. Forme galénique gastrorétentive comprenant un analogue de GABA, au moins un agent gonflant, au moins un retardateur de libération polymère dépendant du pH non gonflant et au moins un excipient pharmaceutiquement acceptable ;
l'analogue de GABA étant choisi entre prégabaline, gabapentine, baclofène, vigabatrine, ((1R,5S)-3-Aminométhyl-1,5-diméthyl-bicyclo[3.2.0]hept-3-yl)-acide acétique ; ((1S,5R)-3-Aminométhyl-1,5-diméthyl-bicyclo[3.2.0]hept-3-yl)-acide acétique ; ((1R,5S)-3-Aminométhyl-6,6-diméthyl-bicyclo[3.1.0]hex-3-yl)-acide acétique ; ((1S,5R)-3- Aminométhyl-6,6-diméthyl-bicyclo[3.1.0]hex-3-yl)-acide acétique ; ((1S,2S,5R)-2-Aminométhyl-6,6-diméthyl-bicyclo[3.1.0]hex-2-yl)-acide acétique ; ((1R,2S,5S)-2-Aminométhyl-6,6-diméthyl-bicyclo[3.1.0]hex-2-yl)-acide acétique ; ((1S,2R,5R)-2-Aminométhyl-6,6-diméthyl-bicyclo[3.1.0]hex-2-yl)-acide acétique ; ((1R,2R,5S)-2-Aminométhyl-6,6-diméthyl-bicyclo[3.1.0]hex-2-yl)-acide acétique ; ((1R,5R,6S)-6-Aminométhyl-bicyclo[3.2.0]hept-6-yl)-acide acétique ; ((1S,5S,6S)-6-Aminométhyl-bicyclo[3.2.0]hept-6-yl)-acide acétique ; ((1R,5R,6R)-6-Aminométhyl-bicyclo[3.2.0]hept-6-yl)-acide acétique ; ((1S,5S,6R)-6-Aminométhyl-bicyclo[3.2.0]hept-6-yl)-acide acétique ; cis-((1 S,2R,4S,5R)-3-Aminométhyl-2,4-diméthyl-bicyclo[3.2.0]hept-3-yl)-acide acétique ; trans-((1 S,2R,4S,5R)-3-Aminométhyl-2,4-diméthyl-bicyclo[3.2.0]hept-3-yl)-acide acétique ; ((1S,5R,6S,7R)-3-Aminométhyl-6,7-diméthyl-bicyclo[3.2.0]hept-3-yl)-acide acétique ; ((1S,5R,6R,7S)-3-Aminométhyl-6,7-diméthyl-bicyclo[3.2.0]hept-3-yl)-acide acétique ; ((1R,2S,5S)-7-Aminométhyl-3,3-diméthyl-tricyclo[3.3.0.0]oct-7-yl)-acide acétique ; ((1R,6R,7S)-7-Aminométhyl-bicyclo[4.2.0]oct-7-yl)-acide acétique ; ((1S,6S,7S)-7-Aminométhyl-bicyclo[4.2.0]oct-7-yl)-acide acétique ; ((1R,6R,7R)-7-Aminométhyl-bicyclo[4.2.0]oct-7-yl)-acide acétique ; ((1S,6S,7R)-7-Aminométhyl-bicyclo[4.2.0]oct-7-yl)-acide acétique ; ((1R,7R,8S)-8-Aminométhyl-bicyclo[5.2.0]non-8-yl)-acide acétique ; ((1S,7S,8S)-8-Aminométhyl-bicyclo[5.2.0]non-8-yl)-acide acétique ; ((1R,7R,8R)-8-Aminométhyl-bicyclo[5.2.0]non-8-yl)-acide acétique ; ((1S,7S,8R)-8-Aminométhyl-bicyclo[5.2.0]non-8-yl)-acide acétique ; [(1R,5R,6S)-6-(Aminométhyl)bicyclo[3.2.0]hept-6-yl]acide acétique ; [(1S,5S,6R)-6-(Aminométhyl)bicyclo[3.2.0]hept-6-yl]acide acétique ; (1RS,5RS,6RS)-6-(Aminométhyl)bicyclo[3.2.0]hept-6-yl]acide acétique ; et [(1RS,6RS,7SR)-7-(Aminométhyl)bicyclo[4.2.0]oct-7-yl]acide acétique ou une combinaison de ceux-ci ;
la quantité d'agent gonflant dans la forme galénique étant comprise entre 5 et 70 % en poids de la forme galénique, et le ou les agents gonflants gonflant en volume en présence de contenu gastrique pour augmenter la taille de la forme galénique de sorte que son passage à travers le pyrole soit empêché ; et
le retardateur de libération polymère dépendant du pH non gonflant étant présent dans la formulation dans une quantité comprise entre 2 et 85 % en poids de la forme galénique et le retardateur de libération polymère dépendant du pH non gonflant étant un polymère d'acide polyméthacrylique ou un dérivé de celui-ci choisi entre coprolymère d'acrylate de méthyle-acide acrylique, coprolymère d'acrylate de méthyle-acide méthacrylique, coprolymère d'acide méthacrylique-méthacrylate de méthyle, coprolymère d'acide méthacrylique-acrylate d'éthyl, copolymère d'acrylate de méthyle-acide méthacrylique-acrylate octylique ou une combinaison de ceux-ci.

2. Forme galénique selon la revendication 1, l'analogue de GABA étant la prégabaline, la gabapentine, le baclofène ou la vigabatrine.

3. Forme galénique selon la revendication 1, l'agent gonflant étant de l'oxyde polyalkylène, polymère cellulosique, polymère d'acide acrylique, polymère d'anhydride maléique ; acide polymaléique, poly(acrylamide), poly(alcool oléfinique), poly(N-vinyl lactame), polyol, saccharide polyoxyéthylé, polyoxazoline, polyvinylamine, alcool polyvinylique, polyimine, polysaccharide, hydrogel de polyuréthane, zéine, polymère à base de vernis à la gomme-laque, oxyde de polyéthylène, hydroxypropylcellulose, hydroxypropylméthylcellulose, hydroxyéthylcellulose, carboxyméthylcellulose de sodium, carboxyméthylcellulose de calcium, méthylcellulose, acide polyacrylique, gomme de xanthane, et alcool polyvinylique ou dérivé, ou mélange de ceux-ci.

4. Forme galénique selon la revendication 1, l'excipient pharmaceutiquement acceptable étant un améliorant de gonflement, un agent générateur de gaz, un liant, un lubrifiant, un diluant, un délitant, un glissant ou un colorant.

5. Forme galénique selon la revendication 1, la forme galénique étant sous la forme d'un système à double couche ou multicouche ou in-lay.

6. Forme galénique selon la revendication 5, la forme galénique étant sous la forme d'une forme galénique gastrorétentive bicouche.

7. Forme galénique selon la revendication 6, la forme galénique gastrorétentive bicouche comprenant (a) une couche active et (b) une couche gastrorétentive.

8. Forme galénique selon la revendication 7, la couche active comprenant un analogue de GABA, au moins un polymère d'acide polyméthacrylique retardateur de libération polymère dépendant du pH non gonflant ou un dérivé de celui-ci, au moins un excipient pharmaceutiquement acceptable, et éventuellement au moins un agent gonflant ; et la couche gastrorétentive comprenant au moins un agent gonflant et au moins un excipient pharmaceutiquement acceptable.

9. Forme galénique selon la revendication 1, la forme galénique étant conservée dans le tractus gastro-intestinal supérieur pendant une période comprise entre environ 30 minutes et environ 12 heures.

10. Forme galénique selon la revendication 1, la forme galénique libérant l'analogue de GABA sur une période maximale d'environ 24 heures.
